# EUROPEAN PATENT APPLICATION

(11) **EP 3 513 732 A1**
(43) Date of publication of application: **24.07.2019**
(21) Application number: 17862959.8
(22) Date of filing: 04.10.2017
(51) Int. Cl.: A61B 8/00, G01S 15/00, A61M 5/00

(54) **PORTABLE ULTRASOUND DEVICE FOR GUIDING TRANSCUTANEOUS PUNCTURES**

(30) Priority: 17.10.2016 ES 201631334
(71) Applicant: Dispositivos Médicos Flecho, S.L., 30009 Murcia (ES)
(72) Inventor: ANDREU CAYUELAS, José Manuel, 30009 Murcia (ES); GARCÍA BERNÁ, José Alberto, 30009 Murcia (ES); MONTALBÁN LARREA, Salvador, 30009 Murcia (ES)
(74) Representative: Carvajal y Urquijo, Isabel
(86) International application number: PCT/ES2017/070648
(87) International publication number: WO 2018/073467

(57) **Abstract**

The invention relates to a portable ultrasound device for guiding transcutaneous punctures, which comprises a casing (8); a main screen (4), an ultrasound transducer (1) oriented with the ultrasound beam (9) thereof parallel to the main screen (4); an image processing unit (2) that shows the signal of the ultrasound transducer (1) on the main screen (4); means for selecting the scale of amplification of the ultrasound image shown on the main screen (4); a width reference system (12) located in the front lower part of the casing (8), which includes vertical reference lines (15); and a scale adjustment zone (5) located above the width reference system (12) and wherein the image processing unit (2) shows scale adaptation lines (16) with an inclination that varies depending on the scale selected. The reference system can be printed on the casing (8) itself, on the main screen (4) or on a specific display.

## Description

### Field of the invention

The invention falls within the sector of medical devices for diagnostic imaging, and more specifically ultrasound-based imaging devices for assistance in procedures involving transcutaneous puncturing.

### Background of the invention

The first devices for ultrasound-based medical imaging arose around the middle of the 20th century [1]. The absence of relevant side effects related to the use thereof and, above all, its growing accuracy and speed have exponentially expanded the applications of these devices in the medical and veterinary fields.

The ability to have ultrasonic transducers small enough to fit in one hand, with good spatial resolution and a high image refresh speed have made them a very useful technique for guiding certain invasive procedures with great accuracy. This is the case of procedures for punctures through the skin (or transcutaneous puncture), having an image in almost real time which enables the different soft tissues, blood vessels and nerves to be distinguished, besides being able to discern the trajectory of the needle through them [2] which increases the accuracy with which the punctures are performed and increases safety for the patients.

To do so, the procedures for transcutaneous puncture wherein the use of an ultrasound is recommended to guide the needle have become more numerous in the past few years, including among others the following:
- Cannulation of peripheral veins and arteries [3].
- Cannulation of central veins and arteries [4, 5].
- Muscular/joint infiltration and peripheral nerve block [6].
- Puncture and aspiration and biopsies of subcutaneous injuries, extraction of foreign bodies and guiding in dermatological surgery [7]. (i.e.: cysts, lipomas and nodes).
- Sclerosis of varicose veins.
- Alcoholisation of thyroid lymphadenopathy and other structures.
- Lumbar puncture

One of the puncture procedures with the most consensus regarding the suitability of the use of ultrasound is the cannulation of central venous lines. This technique consists of performing a puncture for introducing catheters into large-calibre veins (mainly internal jugular, axillary, subclavian and femoral veins) and is used to quickly administer a large volume of fluids or medicines, administer parenteral nutrition, monitor haemodynamic parameters and perform a wide variety of diagnostic or therapeutic techniques.

Traditionally, the insertion site of the central venous lines has been determined by means of locating by visualisation and palpation of anatomical structures which have a known spatial relationship with the vein to be cannulated. However, there is evidence that the technique of anatomical references is associated with significant complications, which include arterial puncture, haematoma, pneumothorax, haemothorax, chylothorax, nerve damage or incorrect placement of the catheter among others and which can potentially be lethal [8-11].

There is substantial evidence that the use of ultrasound devices for guiding this type of punctures is able to reduce these complications by 71 %, including the most severe ones such as arterial puncture. Furthermore, it enables the amount of successful procedures to be increased and the number of punctures and the time necessary to conclude the procedure to be reduced [4], which decreases stress for the patient.

These important advantages have caused its indication for use to be accepted by recognised organisations such as the Agency for Healthcare Research and Quality (AHRQ) from the U.S. and the National Institute for Clinical Excellence (NICE) of the British government [12,13], which recommend the routine use thereof in both elective and urgent situations.

However, despite the advantages implied by the use of ultrasound devices for performing these techniques, the use thereof is far from being universal. Some surveys have demonstrated that the adoption of this practice has been low (15-39 %) among pediatric anaesthesiologists, cardiovascular anaesthesiologists and anaesthesiologists of other subspecialities in the United States and Great Britain [14-17], despite the current recommendations from the ARHQ and NICE.

This low use of ultrasound for performing puncture procedures therefore constitutes a public health problem. The causes thereof are explained in part by the features of the ultrasound devices existing on the market. Despite the fact that progressively smaller devices have appeared which improve portability (i.e. General Electric VScan, Philips Lumify, Bard Site-Rite Prevue), until not long ago all the devices had in common an independent transducer which emits and receives the ultrasound, either joined by means of a cable or with wireless connection (for example, Siemens Acuson Freestyle) to a console or another device including the electronic means necessary for reconstructing the image, the screen and the controls, which makes it very difficult to look at the screen and at the surface to be punctured at the same time, complicating the procedure.

In order to solve this problem patent ES2458290-B1, from the same authors as the present invention, discloses a compact, portable ultrasound device locating the screen parallel to the transducer right above the puncture site. However, while this configuration implies advantages with respect to the previous models, the first prototypes of the product have demonstrated significant drawbacks in this arrangement:
- First of all, as mentioned previously, there are multiple uses for ultrasound-guided transcutaneous puncture. Some of these require locating structures with a diameter of just a few millimetres such as veins, nodes or cysts which for the correct visualisation thereof require the image to be amplified. Although locating the screen right above the surface to be punctured improves the visual reference, this is at real scale (without zoom), which is confusing when the image is amplified due to the absence of references with respect to which part of the screen coincides vertically with the anatomical structures reflected thereon.
- Furthermore, it is necessary to highlight that due to ultrasonic physics these are subjected to significant reflection phenomena when they cross interfaces between mediums with different densities and sound transmission speeds. Since there is a large difference in these parameters between air, the transducer itself and the soft tissues of the patient, the use thereof would not be possible if a coupling substance which excludes the air between the ultrasound emitter and the skin of the patient is not used. For this task, a viscous gel with a density similar to that of the soft tissues is usually used which, when applied generously on the lower part of the device, tends to protrude over the front portion of the device which, together with the folds that tend to form in this area of the sterile plastic cover wherein it must be wrapped,can blur the lower part of the screen impeding the correct display of this part of it.

Therefore, despite the evidence of greater effectiveness and safety implied by the use of ultrasound for multiple transcutaneous puncture procedures, the use thereof continues to not be universal due to the difficulty implied by locating the structures to punctured by means of an image on a screen far from the entry point and the difficulty of maintaining sterility in the puncture site with wiring outside of the transducer.

Although a device has been recently proposed which partially resolves these drawbacks, the position of the screen is not optimal since by being at the same level as the ultrasound transducer it creates problems with respect to the visual reference when the ultrasound image is amplified. Furthermore, the display on the lower part of the screen can worsen when the ultrasound gel and the folds in the sterile plastic cover which tend to form in this area get in the way.

### Bibliographic references

1. Dussik, KT. The ultrasonic field as a medical tool. Am J Phys Med Rehabil. 1954; 33(1): 5-20.
2. Hocking G, Hebard S, Mitchell CH. A review of the benefits and pitfalls of phantoms in ultrasound-guided regional anesthesia. Reg Anesth Pain Med. 201;36(2): 162-70.
3. Rabindranath KS, Kumar E, Shail R, Vaux EC. Ultrasound use for the placement of haemodialysis catheters. Cochrane Database Syst Rev. 2011; 11:CD005279.
4. Brass P, Hellmich M, Kolodziej L, Schick G, Smith AF. Ultrasound guidance versus anatomical landmarks for internal jugular vein catheterization..Cochrane Database Syst Rev. 2015;1:CD006962.
5. Brass P, Hellmich M, Kolodziej L, Schick G, Smith AF. Ultrasound guidance versus anatomical landmarks for subclavian or femoral vein catheterization. Cochrane Database Syst Rev. 2015;1:CD011447.
6. Guay J, Suresh S, Kopp S. The use of ultrasound guidance for perioperative neuraxial and peripheral nerve blocks in children. Cochrane Database Syst Rev. 2016;2:CD011436.
7. Mollet-Sánchez J. Ecografia e intervencionismo cutaneo. Actual. Med. 2014; 99: (793). Supl. 57-68.
8. Mansfeld PF, Hohn DC, Fornage BD, et al. Complications and failures of subclavian-vein catheterization. N Engl J Med. 1994; 331:1735-38.
9. Sznajder JI, Zveibil FR, Bitterman H, et al. Central vein catheterization. Failure and complication rates by three percutaneous approaches. Arch Intern Med 1986; 146: 259 - 61.
10. Bernard RW, Stahl WM. Suclavian vein catheterizations: a prospective study. Non infectious complications. Ann Surg. 1971; 173:184-90.
11. Morton PG. Arterial puncture during central venous catheter insertion. CritCare Med. 1999; 27:878-9.
12. Rothschild JM. Ultrasound guidance of central vein catheterization. Evidence Report/Technology Assessment, No. 43. Chapter 21. Making Healthcare Safer. A Critical Analysis of Patient Safety Practices. Agency for Healthcare Research and Quality Publication, No. 01-E058. 2001; 245-253.
13. National Institute for Clinical Excellence, National Health Service. Final appraisal determination: ultrasound locating devices for placing central venous catheters. Available at: http://www.nice.org.uk/guidance/index.jsp?action=article&r=true&o=32460
14. Bosman M., Kavanagh R.H., Two dimensional ultrasound guidance in central venous catheter placement: A survey of pediatric anesthetists in the United Kingdom, Paediatr Anesth. 2006; 16 530-537.
15. Tovey G., Stokes M., A survey of use of 2D ultrasound guidance for insertion of central venous catheters by UK consultant paediatric anesthetists, Eur J Anaesth. 2007; 24 71-75.
16. Bailey P.L., Glance L.G., Eaton M.P. et al., A survey of the use of the ultrasound during central venous catheterization, Anesth Analg. 2007; 104, 491-497.
17. T. McGrattan, J. Duffty and J.S. Green et al., A survey of the use of ultrasound guidance in internal jugular venous cannulation, Anaesthesia. 2008; 63, 1222-1225.

### Description of the invention

In order to facilitate the ultrasound-assisted transcutaneous puncture procedures, the present invention attempts to solve the problems described with the ultrasound imaging devices already existing for this purpose.

The present invention relates to a portable ultrasound device for guiding transcutaneous punctures which includes, inside of a casing, an electric battery, a main screen in the front part of the casing, an ultrasound transducer oriented with the ultrasound beam thereof parallel to the main screen, and an image processing unit which adapts the signal of the ultrasound transducer in order to show an ultrasound image on the main screen.

Additionally, the portable ultrasound device comprises means for selecting the scale of amplification of the ultrasound image shown on the main screen; a width reference system located in the front lower part of the casing and which includes vertical reference lines; and a scale adjustment zone located above the width reference system and wherein the image processing unit shows scale adaptation lines with an inclination that varies depending on the scale selected. The portable ultrasound device can also comprise a wireless charging mechanism system.

In one embodiment, the lower ends of the scale adaptation lines are connected to the upper ends of the vertical reference lines. The image processing unit preferably shows vertical lines on the main screen superimposed over the ultrasound image and which continue the scale adaptation lines, such that the width between consecutive vertical lines depends on the scale selected. The vertical reference lines can represent between the ends thereof the width of the ultrasound beam.

In one possible embodiment, the means for selecting the scale comprise at least one push button located on the outer portion of the device. In another alternative embodiment, the main screen is a touch screen and the means for selecting the scale are implemented by means of said touch screen.

The lower edge of the ultrasound transducer is preferably located in the internal lower part of the casing. The lower edge of the main screen can be located above the lower surface of the ultrasound transducer.

According to a possible embodiment, the scale adjustment zone is implemented by means of a second screen, independent of the main screen, and which is located between the width reference system and the main screen. The second screen can be implemented, for example, by means of an LCD display, an LED display or an e-ink display.

In another possible embodiment, the scale adjustment zone is a lower region of the main screen itself. The width reference system is represented on the end lower region of the main screen itself. Alternatively, the width reference system can be printed on the casing itself.

The portable ultrasound device of the present invention eliminates all external wiring, including the ultrasonic transducer, the image processing system, the battery and the screen inside of a single casing. The position of the screen is very close to the lower surface of the device (preferably between 5 and 50 millimetres above it), which coincides with that of the ultrasound transducer, which enables the image to be displayed better than if it were at the same level. Furthermore, on the lower front surface, just above the surface of the transducer, there is a reference marked with centimetres in the form of vertical marks and between it and the screen wherein the image is shown, there is a "scale zone" or "scale changing zone" able to show lines with different inclination based on the increase selected in order to show the anatomical image. This "scaling zone" is either an independent LCD, LED or e-ink display, or a zone distinguished exclusively for this purpose of the same screen which shows the image reconstructed starting from the ultrasound emitted by the device. In an alternative arrangement, this "scaling zone" can include the vertical lines described on the lower part of the device.

This arrangement enables a clear reference to be obtained of the actual location of the anatomical structures located immediately below the device, even including when zoom is applied to the image. With these modifications, the usefulness of the device is able to be increased and the learning curve necessary to use it is decreased.

### Brief description of the drawings

What follows is a very brief description of a series of drawings that aid in better understanding the invention, and which are expressly related to one embodiment of said invention which is presented by way of nonlimiting examples of the same.
Figure 1 shows a schematic diagram of a possible embodiment of the ultrasound device of the present invention.
Figure 2 shows an exploded view of the device with the components thereof.
Figure 3 shows a possible embodiment of the portable ultrasound of the invention in the position of use.
Figures 4A-4C show an example of the usefulness of the vertical lines on the lower part of the device for the reference when performing a puncture thereunder.
Figure 5 shows an embodiment of the device wherein the width of the ultrasound beam is fairly reduced with respect to the width of the screen.

### Detailed description of the invention

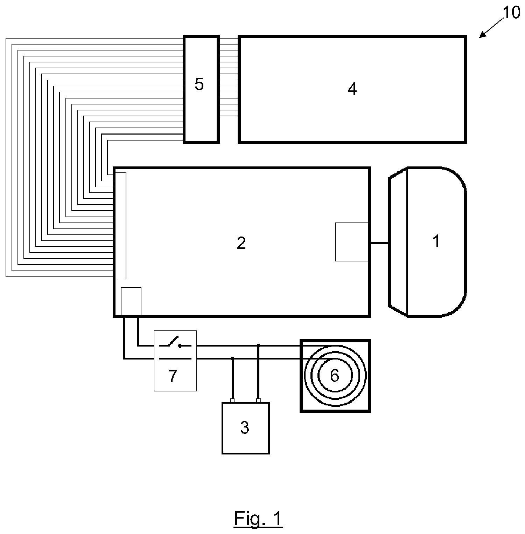

**Figure 1** schematically shows the different elements of the portable ultrasound device 10 according to a possible embodiment. The portable ultrasound device 10 includes, inside a casing (not shown in the figure), an ultrasound transducer 1, an image processing unit 2, en electric battery 3 and a main screen 4, without external wiring. The image processing unit 2 can be implemented, for example by means of a CPU, a GPU or a combination of both.

For displaying the zone of interest with the portable ultrasound device 10, the image processing unit 2 adapts the signal of the ultrasound transducer 1 so that the ultrasound image is shown on the main screen 4. The device shown in Figure 1 includes a second screen or scaling zone or scale adjustment zone 5 which helps to maintain the region of interest located, although the scale adjustment zone 5 could be integrated in the main screen 4 itself. Meaning, the image to be represented in the scale adjustment zone 5 can be presented on the lower end of the main screen 4, or on a second screen located underneath the main screen 4 (considering the orientation of the main screen 4 in the vertical position of use).

For the operations thereof, the ultrasound transducer 1 and the two screens (4, 5) are connected to the image processing unit 2. This connection makes the transit of energy and the flow of data possible. Furthermore, the electric battery 3 makes the device 10 operate autonomously. The device 10 can optionally have a wireless charging system 6 which enables, for example by means of Qi or PMA standards, recharging the battery 3 without needing external wiring, which makes the outer casing able to be more airtight. The presence of a switch 7 enables the device 10 to be turned on and off.

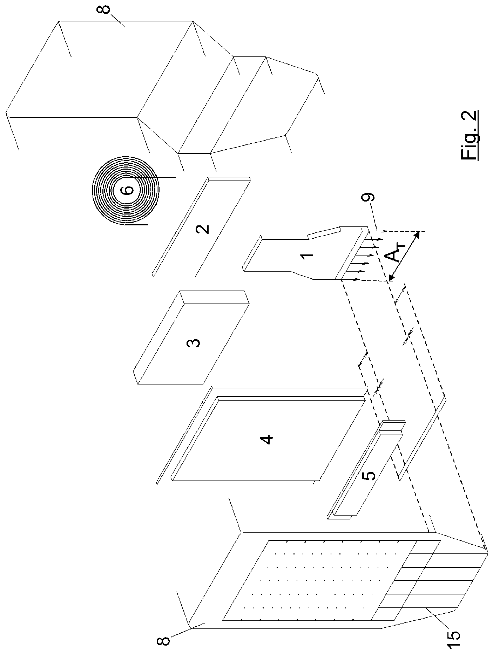

**Figure 2** shows an exploded view of the device 10 with the different components thereof and the relative position between them. Besides the components shown in Figure 1, the casing 8 of the device and the ultrasound beam 9, with width A_{T}, emitted by the ultrasound transducer 1 are also seen. The scale adjustment zone 5 is implemented in a second screen located beneath the main screen 4. The lower front face of the casing 8 includes a width reference system 12 by means of printed vertical reference lines 15, the usefulness of which will be explained further on. As seen in Figure 2, the lower front edge of the casing 8 substantially coincides in height (with the necessary differences due to the thickness of the lower face of the casing 8) with the lower edge of the ultrasound transducer 1.

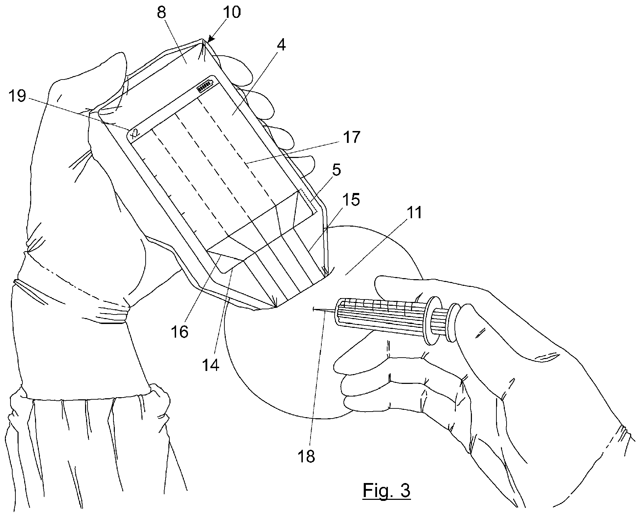

As seen in **Figure 3****,** the portable ultrasound device 10 is designed to be held vertically over the cutaneous surface 11 of the patient, the ultrasound transducer 1 staying in the lower part and the main screen 4 on the front part thereof (facing towards the operator of the device), with the lower edge thereof 14 close to the lower surface of the transducer. The portable ultrasound device 10 shows on the main screen 4 the ultrasound image captured by the ultrasound transducer 1 (in Figure 3 for simplicity only vertical reference lines 17 are shown, not the ultrasound image), wherein the needle 18 inserted in the cutaneous surface 11 of the patient and the vein to be cannulated can be seen.

On the front lower edge of the casing 8, right above the surface of the transducer, reference lines 15 (shown with solid or dotted lines) corresponding to the actual measurements of the transducer which is underneath them are printed, and between these reference lines 15 and the main screen 4, there is a differentiated scale adjustment zone 5 which can be implemented in a second screen (i.e. LCD, LED or e-ink display), or even a distinguished zone of the main screen 4 itself. The reference lines 15 are preferably represented with a plurality of vertical lines, with a constant separation Aᵣ between them and a separation between the ends corresponding to the width A_{T} of the ultrasound beam 9.

In an alternative embodiment, the main screen 4 itself can be extended to the front lower edge of the casing 8, such that the reference lines 15, instead of being engraved or printed on the casing 8, are shown on the main screen 4, which would also encompass the scale adjustment zone 5.

In the scale adjustment zone 5, scale adaptation lines 16 are shown which improve the visual reference of what stays vertical with respect to what is shown in the image. To do so, the lower ends of the scale adaptation lines 16 connect to the upper ends of the vertical reference lines 15 by way of reference, with the aim of determining the vertical line of what is shown on the main screen 4 in order to perform the puncture. The scale adaptation lines 16 of the scale adjustment zone 5, which are normally oblique lines when the image on the screen is amplified, are continued with vertical lines 17 on the main screen 4, shown as solid or dotted lines. This arrangement enables an exact reference of the actual location of the anatomical structures shown on the screen, whether with an image with a 1:1 scale or with an amplification of the image.

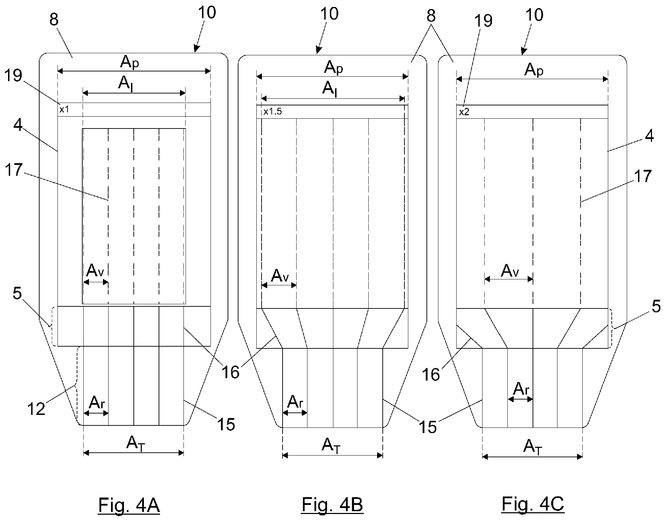

**Figures 4A, 4B and 4C** illustrate the use of different zoom scales 19 to help better understand the usefulness of the scale adjustment zone 5. In the case shown in these figures, the reference lines 15 are printed on the lower part of the casing 8 of the device, and the scale adjustment zone 5 is not implemented as a separate screen, rather it is part of the main screen itself 4 (specifically, the scale adjustment zone 5 occupies a lower region of the main screen 4). The width A of the ultrasound beam 9 corresponds to the width between the end reference lines 15. The zoom scale 19 used in each case can be shown on the main screen 4 for the knowledge of the user of the device (in the example figures, the zoom scales 19 are shown on the left upper edge of the main screen 4).

Figure 4A shows the case of a 1x zoom (in other words, real scale). The reference lines 15 serve to show the actual width of the transducer or width A_{T} of the ultrasound beam 9, which coincides with the scanned zone of the cutaneous surface 11 of the patient at real scale and to be able to convert the dimensions of the actual width of the transducer (width A_{T} of the ultrasound beam 9) to the width of the images shown on the screen. Said conversion is performed in the scale adjustment zone 5, wherein the actual dimensions are converted to the virtual dimensions shown on the screen. Thus, if the image shown on the main screen 4 is not amplified, which is the case of Figure 4A, the width Aᵣ existing between consecutive reference lines 15 coincides with the width Aᵥ between consecutive vertical lines 17, and the width A_{T} of the ultrasound beam 9 coincides with the width A_{I} of the ultrasound image displayed on the main screen 4. Since the width A_{P} of the main screen 4 is greater than the width A_{I} of the ultrasound image, the side margins of the main screen 4 which do not show the ultrasound image are shown in black.

When an amplification is performed on the main screen 4 for example 1.5x zoom as shown in Figure 4B, the width Aᵥ between consecutive vertical lines 17 is larger than the width Aᵣ between reference lines 15, and the width A_{I} of the ultrasound image on the screen is larger than the width A_{T} of the ultrasound beam 9. Thus, the ultrasound images of the transducer can be seen amplified and with more detail, and the user of the device responsible for performing the transcutaneous puncture will be much more accurate when guiding the needle 18 or catheter, for example when they hit a vein or artery, furthermore having clearly related the dimensions and positions of the elements shown on the screen (needle, vein, nodes, etc.) with respect to the actual position of the needle 18, wherein the puncture is performed, by being able to use the reference lines 15 on the lower edge of the device 10 as a reference.

In the case shown in Figure 4C, wherein the ultrasound image on the main screen 4 is even further amplified (2x zoom), the width Aᵥ between vertical lines 17 is even greater, and the ultrasound image is shown in all the width of the main screen 4 (in fact, in this case the width A_{I} of the ultrasound image corresponding to the entire width A_{T} of the ultrasound beam 9 does not fit in the entire width Ap of the main screen 4). The zoom control of the screen can be performed in different manners, for example by means of one or several push buttons located on the outside of the casing 8 or even by means of touch commands or instructions in the case of the main screen 4 being a touch screen, as is the case in the examples shown in Figures 4A-4C.

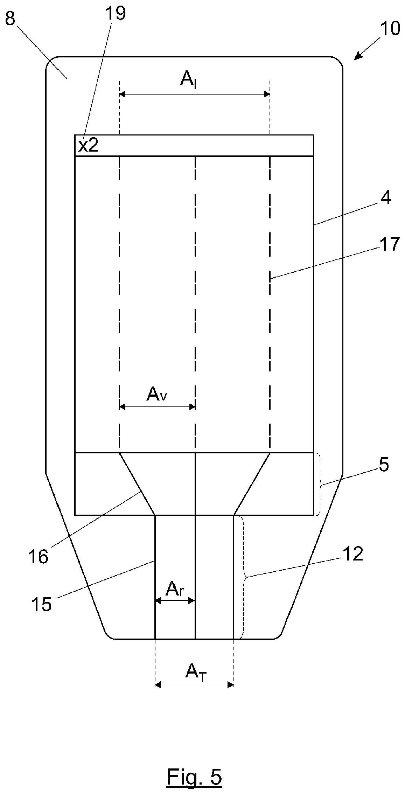

**Figure 5** shows another additional advantage of the present invention. The assembly formed by the reference lines 15, the scale adjustment zone 5 and the vertical lines 17 on the main screen, together with the zoom scale 19 used, works as an adapter or converter of the width A_{T} of the ultrasound beam 9 to the width A_{I} of the ultrasound image shown on the main screen 4. Thus, the portable ultrasound device 10 enables commercial ultrasound transducers 1 with different widths to be used, being independent from the width A_{T} of the ultrasound beam 9 since the device 10 enables any width A_{T} of the ultrasound beam 9 to be converted to the width A_{I} of the suitable ultrasound image so that the user may view it with the amplification and detail considered appropriate, using the appropriate zoom scale 19.

In order to maintain the necessary asepsis in the transcutaneous puncture procedure, the device can be covered with a transparent sterile wrapping which enables the screen to be viewed during the puncture. Furthermore, in order to prevent the existence of charging ports in the casing 8 which can hinder the disinfection of the device 10, a wireless charging system 6 can be included inside the casing 8, as shown in Figures 1 and 2.

## Claims

1. A portable ultrasound device for guiding transcutaneous punctures which includes, inside of a casing (8), an electric battery (3), a main screen (4) in the front part of the casing (8), an ultrasound transducer (1) oriented with the ultrasound beam (9) thereof parallel to the main screen (4), and an image processing unit (2) which adapts the signal of the ultrasound transducer (1) in order to show an ultrasound image on the main screen (4), **characterised in that** the portable ultrasound device (10) additionally comprises:
- means for selecting the scale of amplification of the ultrasound image shown on the main screen (4);
- a width reference system (12) located in the front lower part of the casing (8) and which includes vertical reference lines (15);
- a scale adjustment zone (5) located above the width reference system (12) and wherein the image processing unit (2) shows scale adaptation lines (16) with an inclination that varies depending on the scale selected.

2. The portable ultrasound device according to claim 1, **characterised in that** the lower ends of the scale adaptation lines (16) are connected to the upper ends of the vertical reference lines (15).

3. The portable ultrasound device according to any of the preceding claims, **characterised in that** the image processing unit (2) preferably shows vertical lines (17) on the main screen (4) superimposed over the ultrasound image and which continue the scale adaptation lines (16), such that the width (Aᵥ) between consecutive vertical lines (17) depends on the scale selected.

4. The portable ultrasound device according to any of the preceding claims, **characterised in that** the vertical reference lines (15) represent between the ends thereof the width (A_{T}) of the ultrasound beam (9).

5. The portable ultrasound device according to any of claims 1 to 4, **characterised in that** the means for selecting the scale comprise at least one push button located on the outer portion of the device (10).

6. The portable ultrasound device according to any of claims 1 to 4, **characterised in that** the main screen (4) is a touch screen and the means for selecting the scale are implemented by means of said touch screen.

7. The portable ultrasound device according to any of the preceding claims, **characterised in that** the lower edge of the ultrasound transducer (1) is located in the internal lower part of the casing (8).

8. The portable ultrasound device according to any of the preceding claims, **characterised in that** the lower edge of the main screen (4) is located above the lower surface of the ultrasound transducer (1).

9. The portable ultrasound device according to any of claims 1 to 8, **characterised in that** the scale adjustment zone (5) is implemented by means of a second screen, independent of the main screen (4), and which is located between the width reference system (12) and the main screen (4).

10. The portable ultrasound device according to claim 9, **characterised in that** the second screen (5) is implemented by means of an LCD display, an LED display or an e-ink display.

11. The portable ultrasound device according to any of claims 1 to 8, **characterised in that** the scale adjustment zone (5) is a lower region of the main screen (4) itself.

12. The portable ultrasound device according to claim 11, **characterised in that** the width reference system (12) is represented on the end lower region of the main screen (4) itself.

13. The portable ultrasound device according to any of claims 1 to 11, **characterised in that** the width reference system (12) is printed on the casing (8).

14. The portable ultrasound device according to any of the preceding claims, **characterised in that** it comprises a wireless charging mechanism (6).
